Europäisches Patentamt

European Patent Office

Office européen des brevets

(11) Veröffentlichungsnummer: **0 096 242**
A2

(12) **EUROPÄISCHE PATENTANMELDUNG**

(21) Anmeldenummer: **83104814.5**

(22) Anmeldetag: **16.05.83**

(51) Int. Cl.³: **A 61 B 6/02**
**A 61 B 6/00**

(30) Priorität: **04.06.82 DE 3221179**

(43) Veröffentlichungstag der Anmeldung:
**21.12.83 Patentblatt 83/51**

(84) Benannte Vertragsstaaten:
**DE FR GB**

(71) Anmelder: **SIEMENS AKTIENGESELLSCHAFT**
**Berlin und München Wittelsbacherplatz 2**
**D-8000 München 2(DE)**

(72) Erfinder: **Pfeiler, Manfred, Dr.**
**Ludwig-Thoma-Strasse 25**
**D-8520 Erlangen(DE)**

(72) Erfinder: **Kalender, Willi, Dr.**
**Neue Strasse 4**
**D-8521 Kleinseebach(DE)**

(72) Erfinder: **Linke, Gerhard**
**Ringstrasse 34**
**D-8521 Uttenreuth(DE)**

(54) Schichtaufnahmegerät zur Herstellung von Transversalschichtbildern.

(57) Die Erfindung betrifft einen Computertomographen, der Mittel (10) zum Verriegeln der Meßanordnung (1, 5) gegen Drehung während der Verarbeitung der vom Strahlenempfänger (3) gelieferten Meßsignale und weitere Mittel (8) zum Verriegeln der Patientenliege (6) gegen Längsverschiebung während der Verarbeitung der Meßsignale aufweist. Ferner ist eine verstellbare Blende (2) zum Eingrenzen des Strahlenbündels (3) in der Schichtebene vorgesehen, so daß die Konzentration und/oder Menge eines Röntgenkontrastmittels bei während der Messung stillstehender Meßanordnung und Patientenliege erfaßbar ist. Die Röntgenröhrenspannung wird während der Messung zwischen zwei Werten umgeschaltet, so daß Artefakte weitgehend vermieden sind.

EP 0 096 242 A2

0096242

SIEMENS AKTIENGESELLSCHAFT  Unser Zeichen
Berlin und München  VPA 82 P 3757 E

Schichtaufnahmegerät zur Herstellung von Transversalschichtbildern

Die Erfindung betrifft ein Schichtaufnahmegerät zur
Herstellung von Transversalschichtbildern eines Aufnahmeobjektes, mit einer Patientenliege, einer Strahlenmeßanordnung mit einer Strahlenquelle, die ein das
Aufnahmeobjekt durchdringendes Strahlenbündel erzeugt,
dessen Querschnittsausdehnung senkrecht zur Schichtebene gleich der Schichtstärke ist und mit einem
Strahlenempfänger, der die Strahlungsintensität hinter dem Objekt ermittelt, mit einer Antriebsvorrichtung für die Meßanordnung mit einem Drehgestell zur
Erzeugung von Drehbewegungen der Strahlenmeßanordnung
und mit einem Meßwertumformer für die Transformation
der vom Strahlenempfänger gelieferten Signale in ein
Schichtbild, sowie mit Mitteln zum Verriegeln der
Meßanordnung gegen Drehung während der Verarbeitung
der vom Strahlenempfänger gelieferten Meßsignale, bei
dem Mittel zum Verriegeln der Patientenliege gegen
Längsverschiebung während der Verarbeitung der Meßsignale vorhanden sind und eine verstellbare Blende
zum Eingrenzen des Strahlenbündels in der Schichtebene vorgesehen ist.

Im US-Patent 4.324.978   ist ein Schichtaufnahmegerät
dieser Art beschrieben, bei dem bei gegen Drehung verriegelter Meßanordnung und gegen Längsverschiebung
verriegelter Patientenliege eine Untersuchung der Kontrastmittelkonzentration im durchstrahlten Objektbereich und ihres zeitlichen Verlaufes bei geeigneter

Tp 5 Ler / 19.05.1982

Einblendung des Röntgenstrahlenbündels möglich ist. Es ist ferner bekannt, daß man bei einem Schichtgerät zur Herstellung von Transversalschichtbildern eines Aufnahmeobjektes die Durchstrahlung des Aufnahmeobjektes mit unterschiedlichen Strahlenenergien vornehmen kann und für jede Strahlenenergie den Schwächungskoeffizienten jedes Bildpunktes ermittelt. Daraus können dann die mittlere Ordnungszahl und die Dichte bestimmt werden (DE-AS 23 39 758). Für die Untersuchung des Aufnahmeobjektes mit verschiedenen Strahlenenergien sind bei diesem bekannten Schichtaufnahmegerät zwei Röntgenröhren vorgesehen, die mit unterschiedlichen Spannungen betrieben werden.

Der Erfindung liegt die Aufgabe zugrunde, ein Schichtaufnahmegerät der eingangs genannten Art so auszubilden, daß bei Verwendung einer einzigen Röntgenröhre die Untersuchung der Kontrastmittelkonzentration und ihres Zeitverlaufes mit unterschiedlichen Strahlenenergien vorgenommen werden kann.

Diese Aufgabe ist erfindungsgemäß dadurch gelöst, daß der die als Röntgenröhre ausgebildete Strahlenquelle speisende Röntgengenerator Schaltmittel aufweist, die die Röntgenröhrenspannung während der Messung bei gegen Längsverschiebung verriegelter Patientenliege zwischen zwei Werten umschaltet. Bei dem erfindungsgemässen Schichtaufnahmegerät können die Kontrastmittelkonzentration und der Verlauf des Kontrastmittels bei zwei verschiedenen Röntgenröhrenspannungen verfolgt werden, so daß auch bei Bewegung des Objektes eine Diagnose - wenn keine Untergrundsubtraktion mehr möglich ist - durch Verwendung der mit verschiedenen Spektren gewonnenen Meßdaten möglich ist.

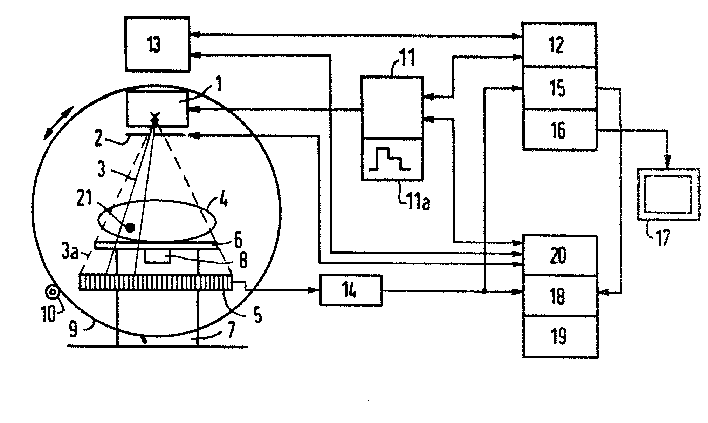

Die Erfindung ist nachfolgend anhand eines in der Zeichnung dargestellten Ausführungsbeispieles näher erläutert.

In der Zeichnung ist eine Röntgenröhre 1 dargestellt, die ein mittels einer Blende 2 eingeblendetes Röntgenstrahlenbündel 3 erzeugt, das einen Patienten 4 in einer Transversalschicht durchdringt. Die Abmessung des Röntgenstrahlenbündels 3 senkrecht zur Schichtebene ist gleich der Schichtstärke. Die aus dem Patienten 4 austretende Röntgenstrahlung trifft auf einem Strahlenempfänger 5 auf, der aus einer Reihe von Detektorelementen besteht und eine solche Abmessung hat, daß er bei vollständiger Bestrahlung, also bei vollständig geöffneter Blende 2, den abzubildenden Querschnitt des Patienten 4 erfaßt. Das Röntgenstrahlenbündel ist für diesen Fall gestrichelt gezeichnet und mit 3a bezeichnet.

Der Patient 4 ruht auf einer Patientenliege 6, die mit einem Sockel 7 verbunden ist und durch eine Verriegelungsvorrichtung 8 gegen Längsverschiebung verriegelt werden kann. Die Meßanordnung aus der Röntgenröhre 1 und dem Strahlenempfänger 5 ist auf einem schematisch dargestellten Drehring 9 angeordnet, der durch einen Antrieb 10 in Pfeilrichtung zur Abtastung des Patienten 4 unter verschiedenen Projektionen gedreht werden kann. Der Antrieb 10 kann den Drehring 9 und damit die Meßanordnung 1, 5 in einer bestimmten Projektion verriegeln.

Die Röntgenröhre 1 wird von einem Röntgengenerator 11 gespeist, der an einer Steuereinrichtung 12 angeschlossen ist, die auch eine Geräteelektronik 13 steuert. Der Strahlenempfänger 5 ist an einer Meßwerterfassungseinheit 14 angeschlossen, die einen Rechner 15 ansteuert, der aus den von der Meßwerterfassungseinrichtung 14 gelieferten Daten ein Bild der untersuchten Transversalschicht des Patienten 4 berechnet. Das berechnete Bild kann in einem Bildspeicher 16 gespeichert und auf einem Sichtgerät 17 optisch wiedergegeben werden. Die Meßdaten der Meßwerterfassungseinrichtung 14 werden zur Bildrekonstruktion während der Drehung der Meßanordnung 1, 5 um einen Winkel von 360° um den Patienten 4 unter verschiedenen Projektionen gewonnen.

Die Meßwerterfassungseinheit 14 ist an einem weiteren Rechner 18 angeschlossen, der für die Bestimmung der Kontrastmittelkonzentration in einem bestimmten Bereich der untersuchten Transversalschicht dient. Die vom Rechner 18 bestimmten Werte können in einem Speicher 19 gespeichert werden. Er arbeitet mit einer Steuereinrichtung 20 zusammen.

Für die Bestimmung der Kontrastmittelkonzentration wird zunächst eine übliche Abtastung des Patienten 4 vorgenommen und mittels des Rechners 15 ein Bild der untersuchten Transversalschicht bestimmt. Dabei kann beispielsweise der Querschnitt eines Blutgefäßes, in dem die Kontrastmittelkonzentration bestimmt werden soll, berechnet werden. Eine entsprechende Information über diesen Querschnitt gibt der Rechner 15 an den Rechner 18. Anschließend wird mittels der Blende 2 das Röntgenstrahlenbündel, das ursprünglich zur Bilderzeugung den gesamten Strahlenempfänger 5 getroffen hat, von seiner Größe 3a auf die Größe 3 eingeblendet. Die Blendeneinstellung erfolgt dabei von der Steuereinrichtung 20, entsprechend dem vom Benutzer eingegebenen Bereich des Strahlenempfängers 5, der die Röntgenstrahlung messen soll. Es durchsetzt die Transversalschicht des Patienten 4 somit nur im Bereich eines Blutgefäßes 21. Ferner wird mittels der Antriebsvorrichtung 10 der Drehring 9 gegen Drehung und die Patientenliege 6 mittels der Verriegelungsvorrichtung 8 gegen Längsverschiebung verriegelt. Bei ruhendem Patienten 4 und ruhender Meßanordnung 1, 5 werden die Meßwerte derjenigen Detektoren, die von dem aus dem Patienten 4 austretenden Röntgenstrahlenbündel 3 getroffen werden, mittels der Meßwerterfassungseinheit 14 erfaßt und dem Rechner 18 zugeführt. Der Rechner 18 kann aus den Meßwerten und der Information über den Querschnitt des Gefäßes 21 die Kontrastmittelkonzentration bestimmen. Ferner ist es möglich, auf diese Weise die Kontrastmittelmenge zu ermitteln. Die vom Rechner 18 ermittelten Daten können z. B. auf einem Bildschirm sowohl graphisch als auch als Zahlenwerte wiedergegeben werden.

Zusätzlich zur Blende 2 kann auch zwischen dem Patienten 4 und dem Strahlenempfänger 5 eine entsprechende Blende angeordnet werden.

Aus der Figur ergibt sich, daß der Röntgengenerator 11 eine Steuervorrichtung 11a enthält, die während einer Messung bei gegen Drehung verriegelter Meßanordnung 1, 3 und gegen Längsverschiebung verriegelter Patientenliege 6 die an der Röntgenröhre 1 liegende Hochspannung zwischen zwei Werten, nämlich einem hohen und einem niedrigen, umschaltet. Dadurch ist erreicht, daß auch bei Bewegung des Aufnahmeobjektes noch eine genaue Bestimmung der Kontrastmittelkonzentration und Kontrastmittelmenge möglich ist.

1 Patentanspruch

1 Figur

Patentanspruch

Schichtaufnahmegerät zur Herstellung von Transversalschichtbildern eines Aufnahmeobjektes (4), mit einer Patientenliege (6), einer Strahlenmeßanordnung (1, 5) mit einer Strahlenquelle (1), die ein das Aufnahmeobjekt durchdringendes Strahlenbündel (3) erzeugt, dessen Querschnittsausdehnung senkrecht zur Schichtebene gleich der Schichtstärke ist und mit einem Strahlenempfänger (5), der die Strahlungsintensität hinter dem Objekt (4) ermittelt, mit einer Antriebsvorrichtung (10) für die Meßanordnung (1, 5) mit einem Drehgestell (9) zur Erzeugung von Drehbewegungen der Strahlenmeßanordnung (1, 5) und mit einem Meßwertumformer (15) für die Transformation der vom Strahlenempfänger (3) gelieferten Signale in ein Schichtbild, sowie mit Mitteln (10) zum Verriegeln der Meßanordnung (1, 5) gegen Drehung während der Verarbeitung der vom Strahlenempfänger (5) gelieferten Meßsignale, bei dem Mittel (8) zum Verriegeln der Patientenliege (6) gegen Längsverschiebung während der Verarbeitung der Meßsignale vorhanden sind und eine verstellbare Blende (2) zum Eingrenzen des Strahlenbündels (3) in der Schichtebene vorgesehen ist, d a d u r c h g e k e n n z e i c h n e t , daß der die als Röntgenröhre ausgebildete Strahlenquelle (1) speisende Röntgengenerator (11) Schaltmittel (11a) aufweist, die die Röntgenröhrenspannung während der Messung bei gegen Drehung verriegelter Meßanordnung (1, 5) und gegen Längsverschiebung verriegelter Patientenliege (6) zwischen zwei Werten umschaltet.